(19) Europäisches Patentamt
European Patent Office
Office européen des brevets

(11) Veröffentlichungsnummer: **0 209 821**
**A2**

(12) # EUROPÄISCHE PATENTANMELDUNG

(21) Anmeldenummer: 86109586.7

(51) Int.Cl.[4]: C 07 D 231/06

(22) Anmeldetag: 14.07.86

(30) Priorität: 24.07.85 DE 3526444

(43) Veröffentlichungstag der Anmeldung:
28.01.87 Patentblatt 87/5

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Seng, Florin, Dr.
Am Katterbach 46
D-5060 Bergisch Gladbach 2(DE)

(72) Erfinder: Schellhammer, Carl-Wolfgang, Dr.
Katharinental 26
D-5060 Bergisch Gladbach 2(DE)

(54) **Pyrazolinverbindungen.**

(57) Optische Aufheller der Formel

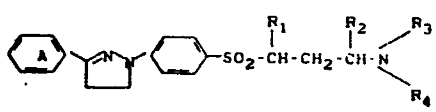

(I)

worin

$R_1$ und $R_2$, H oder $CH_3$, jedoch nicht beides gleichzeitig bedeuten,

$R_3$ und $R_4$ $C_1$-$C_4$-Alkyl bedeuten oder – gemeinsam unter Einschluß des N-Atoms – den Rest eines gesättigten Heterocyclus bilden und der Ring

A durch weitere übliche nichtionische Substituenten substituiert sein kann,

sowie insbesondere deren Quaternierungs- und Protonierungsprodukte zeichnen sich durch hohe Lagerungsbeständigkeit ihrer wäßrigen Lösungen aus.

BAYER AKTIENGESELLSCHAFT 5090 Leverkusen, Bayerwerk
Konzernverwaltung RP
Patentabteilung K/ABc

## Pyrazolinverbindungen

Gegenstand der Erfindung sind Verbindungen der Formel

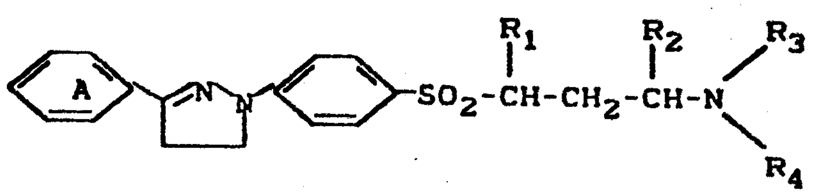

(I)

worin

$R_1$ und $R_2$ Wasserstoff oder Methyl, jedoch nicht beides gleichzeitig bedeuten,

$R_3$ und $R_4$ unabhängig voneinander $C_1$-$C_4$-Alkyl bedeuten oder - gemeinsam unter Einschluß des N-Atoms- den Rest eines gesättigten Heterocyclus bilden und der Ring

A durch weitere in der Chemie der Weißtöner übliche nichtionische Substituenten substituiert sein kann,

sowie deren Quaternierungs- und Protonierungsprodukte.

Geeignete Alkylreste $R_3/R_4$ sind vor allem Methyl und Ethyl. Geeignete Heterocyclen, die $R_3$ und $R_4$ gemeinsam bilden können sind Morpholin- und Piperidinreste.

Unter nichtionischen Substituenten, die der Ring A tragen kann, sind Halogen, $C_1$-$C_4$-Alkyl- und $C_1$-$C_4$-Alkoxygruppen zu verstehen.

Geeignete Quaternierungs- und Protonierungsprodukte sind solche, die man aus den "neutralen" Verbindungen der Formel I durch Behandlung mit in der Farbstoff- oder Aufheller-Chemie üblichen Quaternierungs und Protonierungsmitteln, wie sie z.B. in der DE-OS 2 821 116 oder US-PS 4 051 117 beschrieben sind, erhält.

Bevorzugte Mittel dieser Art sind Dimethylsulfat, Diethylsulfat, Chlorwasserstoffsäure, gegebenenfalls durch Cl oder $CH_3$ substituierte Benzolsulfonsäure, Ethylenoxid, Propylenoxid, Ameisensäure, Essigsäure und Milchsäure.

Bevorzugte Pyrazolinverbindungen sind solche der Formeln

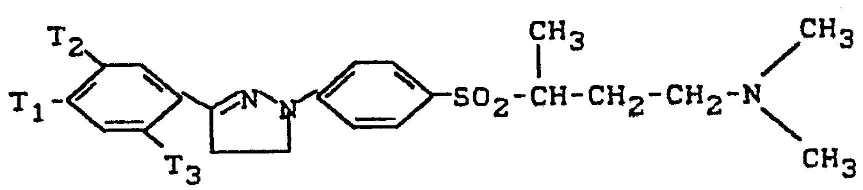

(IIa)

oder

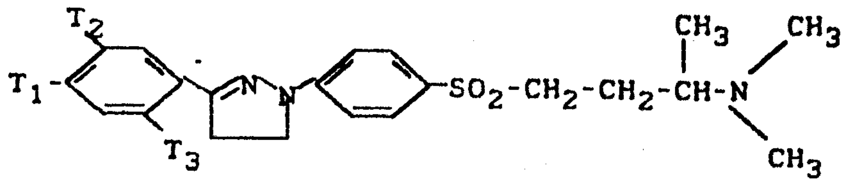 (IIb)

worin

$T_1$ und $T_2$ H oder Cl und

$T_3$ H oder $CH_3$ bedeuten,

sowie deren Quaternierungs- und Protonierungsprodukte.

Die neuen Pyrazolinverbindungen der Formel I, worin $R_2$ = H ist, können beispielsweise dadurch hergestellt werden, daß man in an sich bekannter Weise Ketonverbindungen der Formel

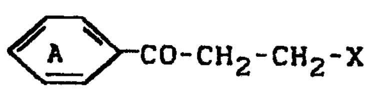 (III)

worin X eine Abgangsgruppe ist, mit Hydrazinverbindungen der Formel

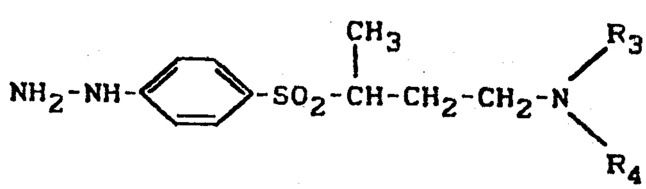 (IV)

umsetzt und das Reaktionsprodukt gewünschtenfalls quaterniert oder protoniert.

Geeignete Abgangsgruppen X sind Halogenatome (vorzugsweise Cl) und Di-$C_1$-$C_4$-alkylaminogruppen.

Die Hydrazinverbindungen der Formel IV erhält man nach folgenden Reaktionsschema:

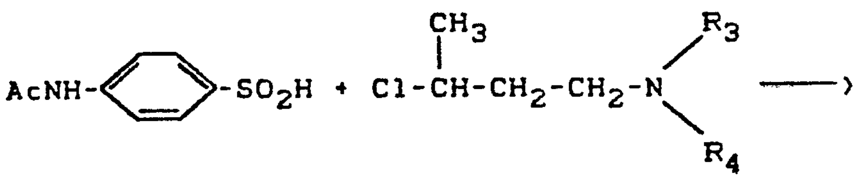

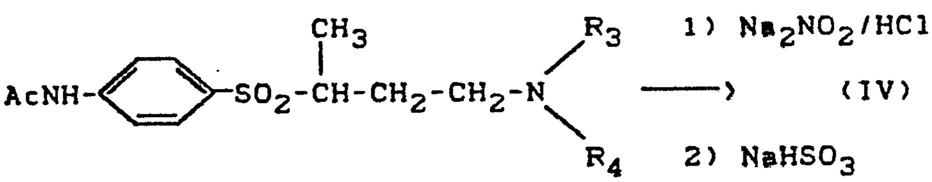


(Ac = Acylrest, z.B. Acetyl)

Die Kondensation der Sulfinsäure mit dem Chloramin erfolgt bei erhöhten Temperaturen (100-200°C) in einem indifferenten Lösungsmittel, z.B. in siedenden Glykolmonomethylether.

Die anschließende Hydrazinsynthese wird unter üblichen Bedingungen durchgeführt.

Die Pyrazolinverbindungen der Formel I, worin $R_1$ = H ist, erhält man nach folgenden Reaktionsschema:

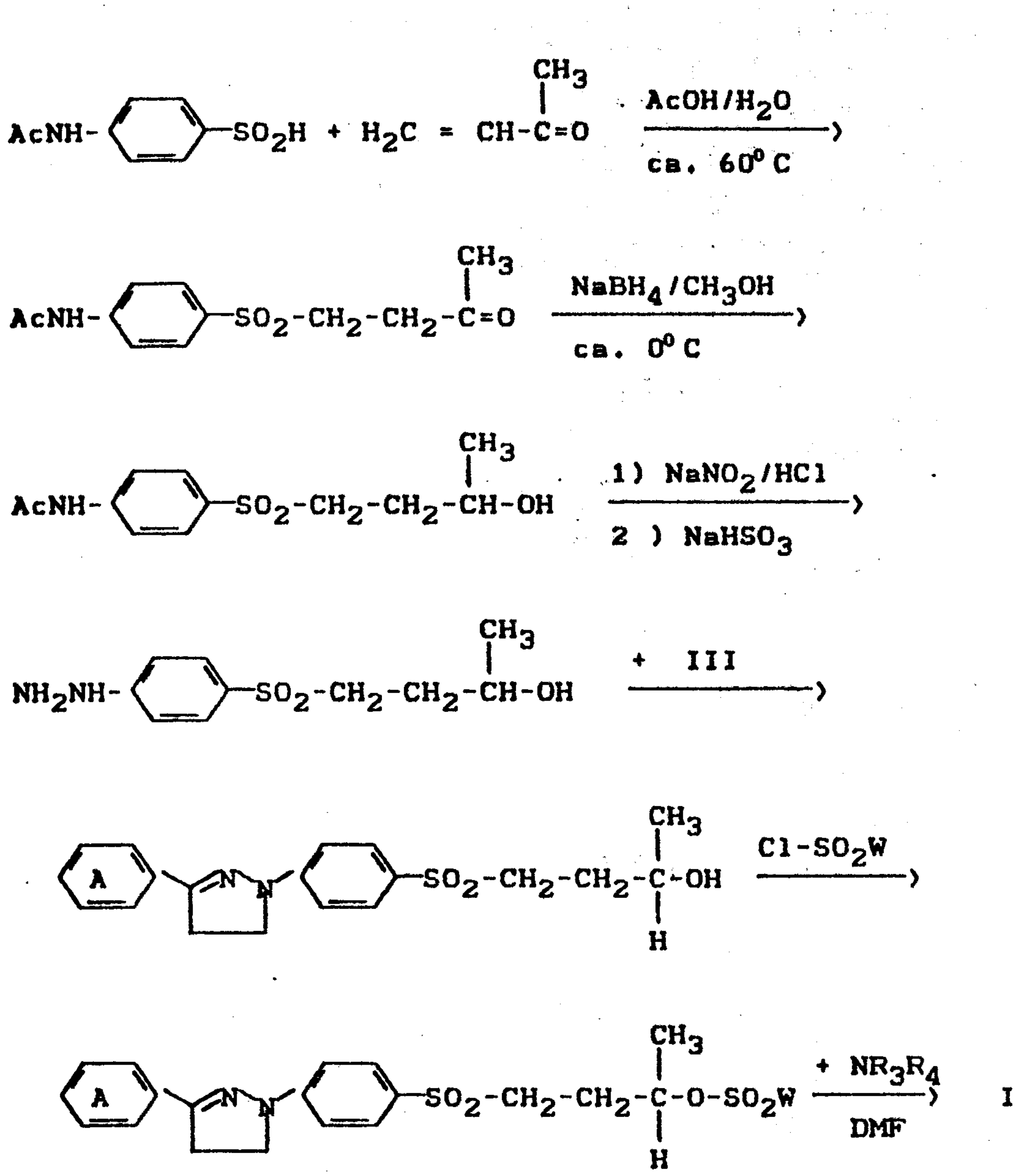


(W = $C_1$-$C_4$-Alkyl)

Die einzelnen Reaktionsschritte werden unter sonst üblichen Bedingungen durchgeführt.

Die neuen Pyrazolinverbindungen zeigen in gelöstem oder feinverteiltem Zustand eine stark Fluoreszenz und eignen sich hervorragend als optische Aufheller. Während die "neutralen" Verbindungen insbesondere zum Weißtönen von Textilmaterialien aus hydrophoben Fasern, insbesondere Polyamidfasern eingesetzt werden, dienen die bevorzugten quaternierten und protonierten Produkte zum Aufhellen von sauer-modifizierten Fasermaterialien, wie z.B. Acrylfasern, nach üblichen Färbemethoden und zum Aufhellen von Spinnmassen.

Diese bevorzugten Weißtöner entsprechend der Formel

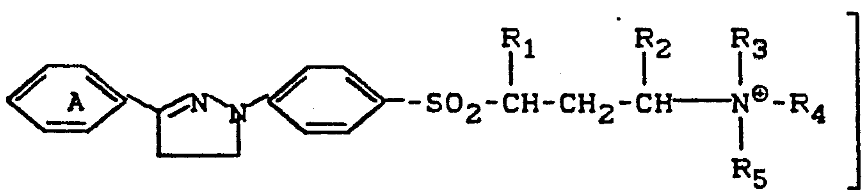 An$^{\ominus}$ (V)

worin

$R_5$ Wasserstoff, $C_1$-$C_4$-Alkyl oder $C_2$-$C_4$-Hydroxyalkyl und

An$^{\ominus}$ ein farbloses Anion, das sich von den obengenannten Quaternierungs- und Protonierungsmitteln ableitet,

bedeutet.

Diese Verbindungen zeichnen sich gegenüber konstitutionell nächstvergleichbaren bekannten Verbindungen, wie sie

z.B. in DE-A 2 700 996 und US-A 3 131 079 beschrieben sind, durch eine höhere Lagerungsbeständigkeit ihrer wäßrigen Lösungen aus.

Beispiel 1

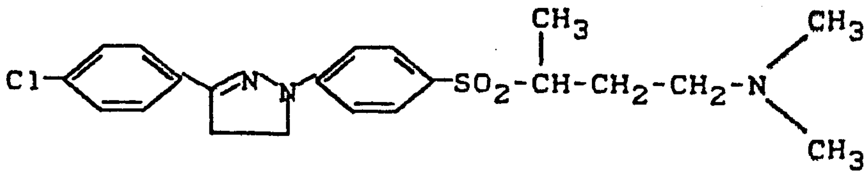

20,3 g (0,1 Mol) 3-Chlor-1-(4-chlorphenyl)-propanon (1) werden zusammen mit 27,1 g (0,1 Mol) 4-(3-Dimethyl-amino-1-methyl-propylsulfonyl)-phenylhydrazin in 150 ml Glykolmonomethylether und 12,5 ml Essigsäure 15 Std. bei 115° gerührt. Anschließend wird das Lösungsmittel bei einer Badtemperatur von 50°C im Wasserstrahlvakuum abdestilliert und der Rückstand in 100 ml Wasser aufgenommen. Die klare Lösung neutralisiert man mit Natriumbicarbonat. Dabei scheiden sich 34,4 g (82 % d.Th.) 1-[4-(3-Dimethylamino-1-methyl-propyl-sulfonylphenyl)]-3-(4-chlorphenyl)-pyrazolin (2) als gelbe Kristalle ab, die nach dem Umlösen aus Ethanol bei 154-6°C schmelzen $\lambda_{max}$ = 368,3 nm (in DMF).

Analog wurden folgende Pyrazoline hergestellt:

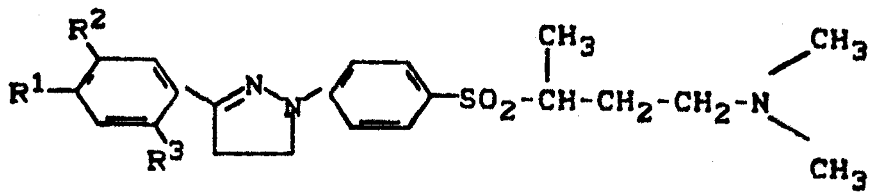

| | $R^1$ | $R^2$ | $R^3$ | Fp[°C] | $\lambda_{max}$ [nm] |
|---|---|---|---|---|---|
| 2) | H | H | H | 122-4 | 362,3 |
| 3) | Cl | Cl | H | 158-60 | 374,4 |
| 4) | Cl | Cl | $CH_3$ | 152-4 | 368,3 |

Beispiel 5

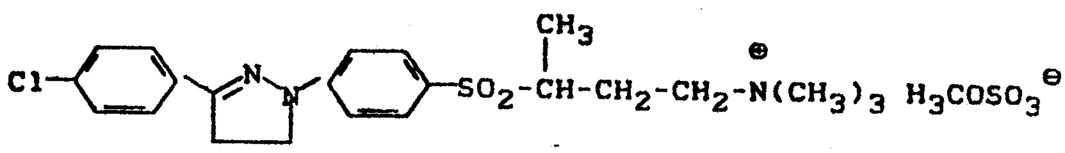

4,2 g (0,01 Mol) 1-[4-(3-Dimethylamino-1-methyl-propyl-sulfonyl-phenyl)]-3-(4-Chlorphenyl)-pyrazolin (2) werden in 70 ml Chlorbenzol gelöst und mit 2 g Dimethylsulfat versetzt. Die Lösung wird sofort trüb, man rührt 20 Std. bei Raumtemperatur und saugt anschließend ab. Man erhält 5,1 g (93,5 % d.Th.) 1-[4-(3-Trimethylammonium-1-methyl-propyl-sulfonyl-phenyl)]-3-(4-chlorphenyl)-pyrazolin-(2)-methosulfat in Form gelber Kristalle, die nach dem Umlösen aus Methanol bei 135°C unter Zersetzung schmelzen. $\lambda_{max}$ = 366,3 nm (in $H_2O$)

Analog wurden folgende Pyrazoline hergestellt:

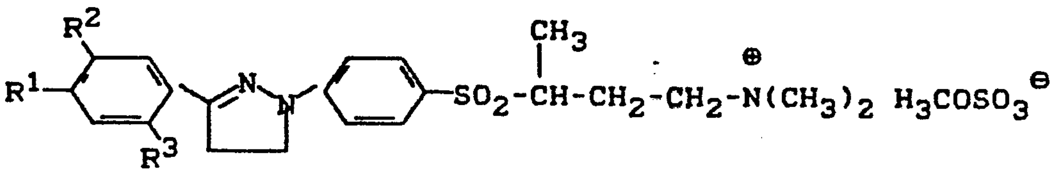

| | $R^1$ | $R^2$ | $R^3$ | Fp[°C] | $\lambda_{max}$ [nm] |
|---|---|---|---|---|---|
| 6) | H | H | H | 142 | 362,9 |
| 7) | Cl | Cl | H | 162-4 | 375,5 |
| 8) | Cl | Cl | $CH_3$ | 262-4 | 368,2 |
| 9) | F | H | H | | |

Beispiel 10

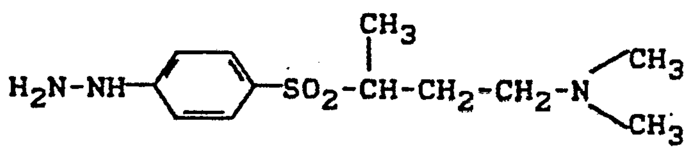

149 g (0,5 Mol) 4-(3-Dimethylamino-1-methyl-propyl-sulfonyl)-acetanilid werden in einer Mischung aus 750 ml 37 %iger Salzsäure und 750 ml Wasser 30 min bei 100°C gerührt. Anschließend wird bei 0 bis 5°C mit 35 g Natriumnitrit, gelöst in 90 ml Wasser diazotiert. Die erhaltene Diazoniumsalzlösung läßt man innerhalb einer Stunde bei 0 bis 8°C in eine Mischung aus 375 ml Wasser und 375 ml 40 %iger Natriumbisulfatlösung fließen, wobei man durch Zugabe von insgesamt 78 g 45 %iger Natronlauge den pH-Wert bei 7 hält. Anschließend rührt man eine Stunde bei Raumtemperatur, gibt dann 175 g 37 %ige Salzsäure zu und rührt

eine weitere Stunde bei 100°C. Zur Entfernung des Schwefeldioxides leitet man während des Abkühlens 4 Stunden lang gasförmigen Stickstoff durch den Ansatz. Danach wird mit 300 g 45 %iger Natronlauge alkalisch gestellt und das dabei abgeschiedene 4-(3-Dimethylamino-1-methyl-propylsulfonyl)-phenylhydrazin in 200 ml Diethylether aufgenommen. Nach dem Trocknen über Natriumsulfat und Abdestillation des Ethers hinterbleiben 110 g Hydrazin als gelbbraunes Öl.

Beispiel 11

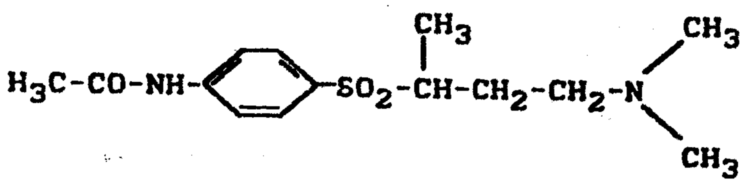

Eine Mischung aus 45,6 g (0,23 Mol) 4-Acetyl-amino-benzolsulfinsäure, 22 g Natriumbicarbonat und 32 g (0,23 Mol) 1-Dimethylamino-3-chlorbutan in 180 ml Glykolmonomethylether und 25 ml Wasser wird 6 Stunden bei 115°C gerührt. Anschließend wird das Lösungsmittel im Wasserstrahlvakuum abdestilliert und der Rückstand in 200 ml Wasser gelöst. Beim Versetzten mit 25 g 45 %iger Natronlauge scheiden sich 53 g 4-(3-Dimethylamino-1-methyl-propylsulfonyl)-acetanilid als weiße Kristalle ab, die bei 82-4°C schmelzen.

Beispiel 12

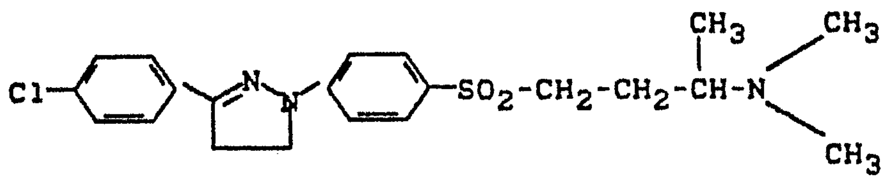

In eine Lösung von 8,5 g (0,02 Mol) 1-[4-(3-Methylsulfonyloxy-butylsulfonyl-phenyl)]-3-(4-chlorphenyl)-pyrazolin (2) in 100 ml Dimethylformamid wird 3 Stunden bei 80° gasförmiges Dimethylamin eingeleitet. Anschließend wird das Dimethylformamid im Wasserstrahlvakuum abdestilliert und der hinterbleibende feste Rückstand aus 120 ml Ethanol umgelöst. Man erhält 5,2 g 1-[4-[3-Dimethylamino-butylsulfonyl-phenyl)]-3-(4-chlorphenyl)-pyrazolin (2) als gelbe Kristalle, die bei 155-7°C schmelzen $\lambda_{max}$: 368,6 nm (DMF).

Analog wurden hergestellt:

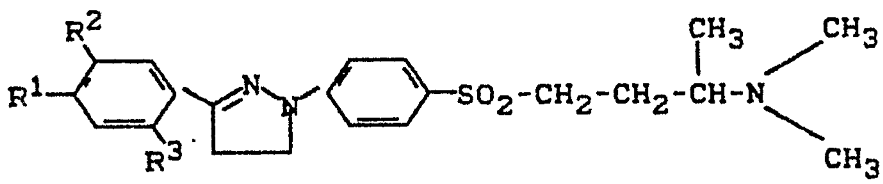

| | $R^1$ | $R^2$ | $R^3$ | Fp[°C] | $\lambda_{max}$ [nm] |
|---|---|---|---|---|---|
| | H | H | H | 118 | 362 |
| 14) | Cl | Cl | H | 168-70 | 375,2 |
| 15) | Cl | Cl | $CH_3$ | 138-40 | 368,5 |

Beispiel 16

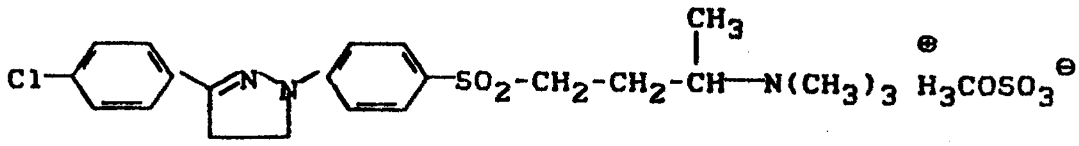

2,1 g (0,05 Mol) 1-[4-(3-Dimethylamino-butylsulfonyl-phenyl)]-3-(4-chlorphenyl)-pyrazolin (2) werden in einer Mischung aus 120 ml Chlorbenzol und 20 ml Dimethylformamid bei 60° C gelöst und mit 1 ml Dimethylsulfat versetzt. Man läßt 12 Std. ohne Heizung rühren und saugt dann die ausgefallenen Kristalle von 1-[4-(3-Trimethylammonium-butylsulfonyl-phenyl)]-3-(4-chlorphenyl)-pyrazolin (2)-methosulfat ab. Ausbeute: 2,2 g, Fp: 155-8, $\lambda_{max}$: 366,2 ($H_2O$)

Analog wurden hergestellt:

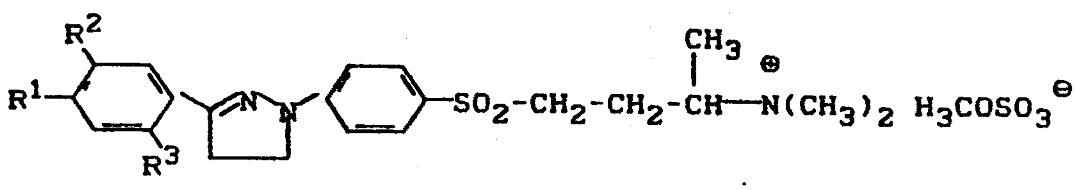

| | $R^1$ | $R^2$ | $R^3$ | Fp[° C] | $\lambda_{max}$ [nm] |
|---|---|---|---|---|---|
| 17) | H | H | H | 146 | 362,9 |
| 18) | Cl | Cl | H | 178-80 | 374,6 |
| 19) | Cl | Cl | $CH_3$ | 260-2 | 368,7 |

Beispiel 20

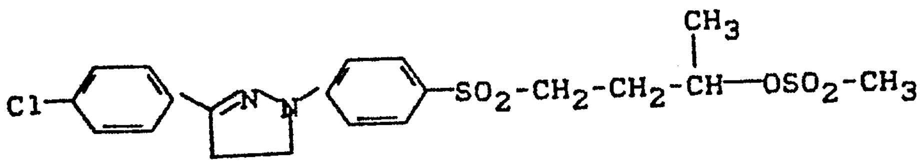

27,3 g (0,08 Mol) 1-[4-(3-Hydroxy-butylsulfonyl-phenyl)]-3-(4-chlorphenyl)-pyrazolin (2) werden in 100 ml Pyridin gelöst und bei Raumtemperatur tropfenweise mit 9,2 g (0,08 Mol) Methansulfonsäurechlorid versetzt. Man rührt 2 Std. bei Raumtemperatur nach und gießt anschließend auf 1 l Wasser. Das ausgefallene Material wird abgesaugt und aus 100 ml Acetonitril umkristallisiert. Man erhält 28,3 g 1-[4-(3-Methylsulfonyloxy-butylsulfonyl-phenyl)]-3-(4-chlorphenyl)pyrazolin (2) als gelbe Kristalle die bei 171-4° schmelzen.

Analog wurden hergestellt:

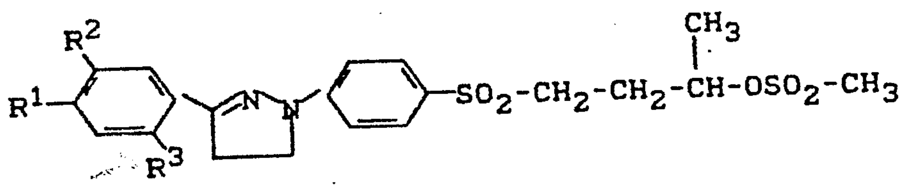

| | $R^1$ | $R^2$ | $R^3$ | Fp [° C] |
|---|---|---|---|---|
| 21) | H | H | H | 104-8 |
| 22) | Cl | Cl | H | 168-70 |
| 23) | Cl | Cl | $CH_3$ | 170-3 |

Beispiel 24

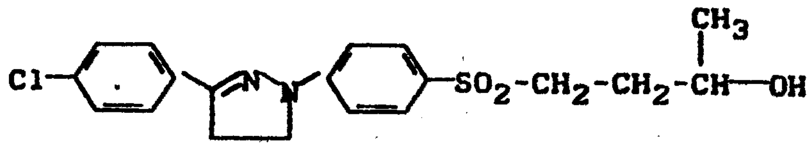

20,3 g (0,1 Mol) 3-Chlor-1-(4-chlorphenyl)-propanon (1) werden zusammen mit 24,4 g (0,1 Mol) 4-(3-Hydroxybutyl-sulfonyl)-phenylhydrazin in 150 ml Glykolmonomethylether und 10 ml Essigsäure 3,5 Std. bei 150° C gerührt. Anschließend wird abgekühlt und abgesaugt. Das Rohprodukt wird aus 150 ml Ethanol umkristallisiert. Dabei erhält man 29,8 g 1-[4-(3-Hydroxy-butylsulfonyl-phenyl)]-3-(4-chlorphenyl)-pyrazolin (2) als gelbe Kristalle, die bei 168-9° C schmelzen.

Analog wurden hergestellt:

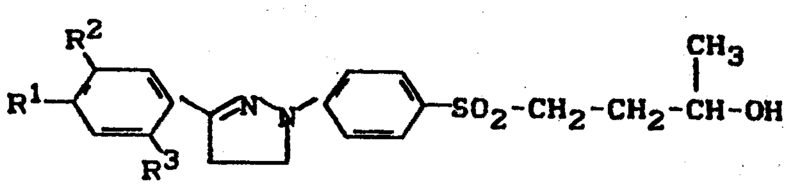

| | $R^1$ | $R^2$ | $R^3$ | Fp[° C] |
|---|---|---|---|---|
| 25) | H | H | H | 181-3 |
| 26) | Cl | Cl | H | 148-50 |
| 27) | Cl | Cl | $CH_3$ | 201-2 |

Beispiel 28

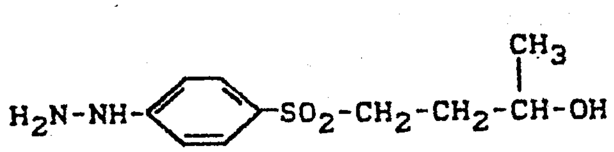

27 g (0,1 Mol) 4-(3-Hydroxy-butylsulfonyl)-acetanilid werden in einer Mischung aus 60 ml 37 %iger Salzsäure und 60 ml Wasser 2 Stunden gekocht. Danach wird die abgekühlte Lösung mit einer Lösung von 6,9 g Natriumnitrit in 15 ml Wasser diazotiert. Die erhaltene Diazoniumsalzlösung läßt man bei 0 bis 5°C in eine Mischung aus 70 ml 40 %iger Natriumbisulfitlösung, 70 ml Wasser und 47 g 45 %iger Natronlauge fließen. Durch gleichzeitige Zugabe von insgesamt 60 g 40 %iger Natronlauge hält man den pH-Wert bei 6. Anschließend werden 70 g 37 %ige Salzsäure zugegeben und 6 Std. zum Sieden erhitzt. Nach dem Abkühlen wird mit Natronlauge alkalisch gestellt und das abgeschiedene Öl in Methylenchlorid aufgenommen. Nach dem Trocknen der Methylenchloridlösung über Natriumsulfat wird das Methylenchlorid abdestilliert. Es hinterbleiben 20 g 4-(3-Hydroxybutylsulfonyl)-phenylhydrazin als gelbbraunes Öl.

Beispiel 29

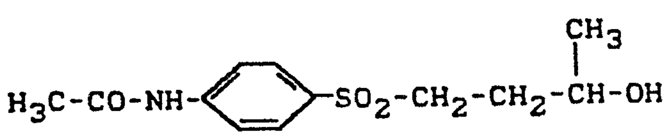

54 g (0,2 Mol) 4-(3-Oxo-butylsulfonyl)-acetanilid werden

in 800 ml Methanol gelöst und innerhalb von 1,5 Std. bei 0°C mit 8,8 g Natriumborhydrid versetzt. Man rührt 6 Std. bei Raumtemperatur nach und säuert dann den Kolbeninhalt mit 10 ml Essigsäure an. Anschließend wird das Methanol im Wasserstrahlvakuum abdestilliert. Der hinterbleibende Rückstand wird in 1 l Essigsäureethylester angerührt. Nach dem Absaugen und Ausrühren des Niederschlages in Wasser hinterbleiben 26 g 4-(3-Hydroxy-butylsulfonyl)-acetanilid als weiße Kristalle die bei 150-2° schmelzen. Aus dem Essigesterfiltrat gewinnt man durch Eindampfen noch weitere 24 g.

Beispiel 30

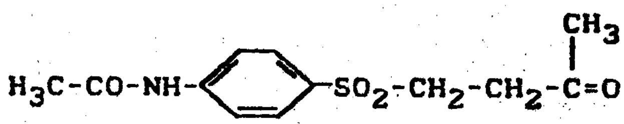

Zu einer Suspension von 10,0 g (0,5 Mol) 4-Acetylamino-benzolsulfinsäure in einer Mischung aus 500 ml Wasser und 100 ml Essigsäure, tropft man bei Raumtemperatur 74 g (0,5 Mol) 94 %iges Butenon (3) und rührt dann 3 Std. bei 60°C nach. Anschließend wird abgekühlt und abgesaugt. Man erhält 137 g 4-(3-Oxo-butylsulfonyl)-acetanilid vom Schmp.: 159-61°.

Patentansprüche

1. Pyrazolinverbindungen der Formel

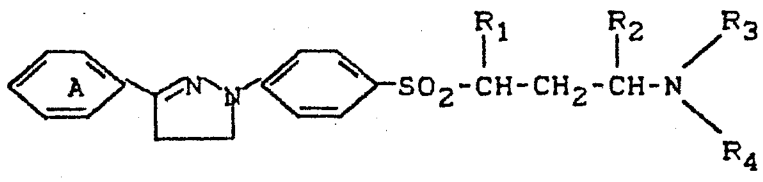

(I)

worin

$R_1$ und $R_2$ Wasserstoff oder Methyl, jedoch nicht beides gleichzeitig bedeuten,

$R_3$ und $R_4$ unabhängig voneinander $C_1$-$C_4$-Alkyl bedeuten oder - gemeinsam unter Einschluß des N-Atoms - den Rest eines gesättigten Heterocyclus bilden und der Ring

A durch weitere in der Chemie der Weißtöner übliche nichtionische Substituenten substituiert sein kann,

sowie deren Quaternierungs- und Protonierungsprodukte.

2. Pyrazolinverbindungen gemäß Anspruch 1 der Formel

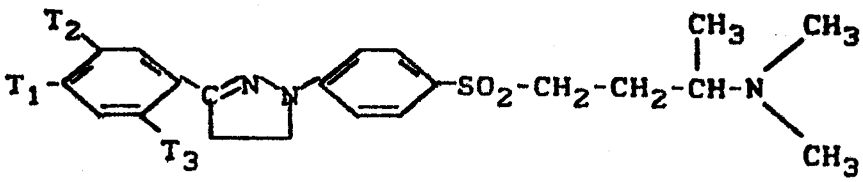

(II)

worin

$T_1$ und $T_2$ H oder Cl und

$T_3$ H oder $CH_3$ bedeuten,

sowie deren Quaternierungs- und Protonierungsprodukte.

3. Pyrazolinverbindungen gemäß Anspruch 1 der Formel

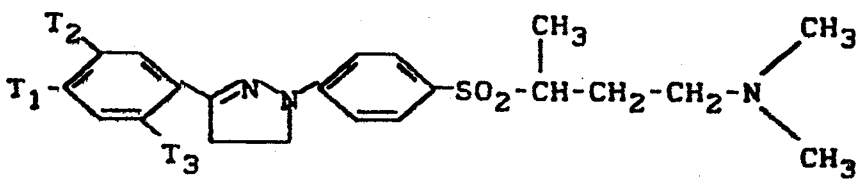

(IIa)

worin

$T_1$ - $T_3$ die in Anspruch 2 genannte Bedeutung haben.